# EUROPEAN PATENT APPLICATION

(11) **EP 4 227 421 A1**
(43) Date of publication of application: **16.08.2023**
(21) Application number: 21877696.1
(22) Date of filing: 07.10.2021
(51) Int. Cl.: C12Q 1/02, G01N 33/53, G01N 33/569

(54) **METHOD FOR DETERMINING PRESENCE/ABSENCE OF NOROVIRUS INFECTION RISK**

(30) Priority: 07.10.2020 JP 2020169704
(71) Applicant: Niigata University, Niigata-shi, Niigata 950-2181 (JP); Denka Company Limited, Tokyo 103-8338 (JP)
(72) Inventor: SAITO, Akihiko, Niigata-shi, Niigata 951-8510 (JP); HOSOJIMA, Michihiro, Niigata-shi, Niigata 951-8510 (JP); KABASAWA, Hideyuki, Niigata-shi, Niigata 951-8510 (JP); AOKI, Nobumasa, Niigata-shi, Niigata 951-8520 (JP); NAGANO, Kei, Niigata-shi, Niigata 951-8520 (JP); SEKINE, Sakari, Tokyo 103-8338 (JP); MUNEKATA, Keisuke, Tokyo 103-8338 (JP)
(74) Representative: MacLean, Martin Robert
(86) International application number: PCT/JP2021/037090
(87) International publication number: WO 2022/075397

(57) **Abstract**

The present invention provides a method of assisting a determination of whether a subject is susceptible to infection by norovirus or whether a subject is susceptible to post-infection worsening, wherein this method contains a step of measuring the concentration of IgG antibody that is present in a sample originating from within the oral cavity of the subject.

## Description

### Technical Field

The present invention relates to, inter alia, broadly a method for determining the presence/absence of a risk of norovirus infection and specifically a method for assisting in the determination of whether a subject is susceptible to norovirus infection or whether a subject is susceptible to a worsening of the disease once infected.

### Background Art

Norovirus is an important virus that causes infectious gastroenteritis. Norovirus is a virus that causes acute gastroenteritis in all age groups from infants to the elderly. The main noroviruses that infect humans are classified into two genogroups, i.e., genogroup I (GI) and genogroup II (GII); 19 types occur in GI and 22 types occur in GII. According to the Center for Surveillance, Immunization, and Epidemiologic Research of the National Institute of Infectious Diseases of Japan, GII is the cause of the majority of the prevalence of infectious gastroenteritis in Japan.

The main symptoms of norovirus infection are diarrhea due to inflammatory reactions in the small intestine, and vomiting and nausea due to the paralysis, or decreased reactivity, of the motor nerves that convey the gastric contents to the intestine; however, the fever is mild. These symptoms continue for 1 to 3 days and then resolve. There are no drugs that are effective against norovirus infection and onset, and only symptomatic treatment, such as rehydration, is available. In addition, a norovirus vaccine, e.g., for preventing infection, has yet to be developed.

Clarification of the factors important in preventing the onset and worsening of norovirus infection is important for, e.g., epidemiological investigations of norovirus and research on norovirus vaccines. An index for assessing the efficacy of a vaccine is essential for research in the area of vaccine development.

### Citation List

### Non-Patent Document

Non-Patent Document 1: J Infect Dis, 2020 Jan 20, pii: jiaa021. Humoral and Mucosal Immuno Responses to Human Norovirus in the Elderly.
Non-Patent Document 2: Clin Diagn Lab Immunl. 2004 Nov, 11(6): 1028-1034 Diagnosis of Norwalk Virus Infection by Indirect Enzyme Immunoassay Detection of Salivary Antibodies to Recombinant Norwalk Virus Antigen.

### Summary

### Technical Problem

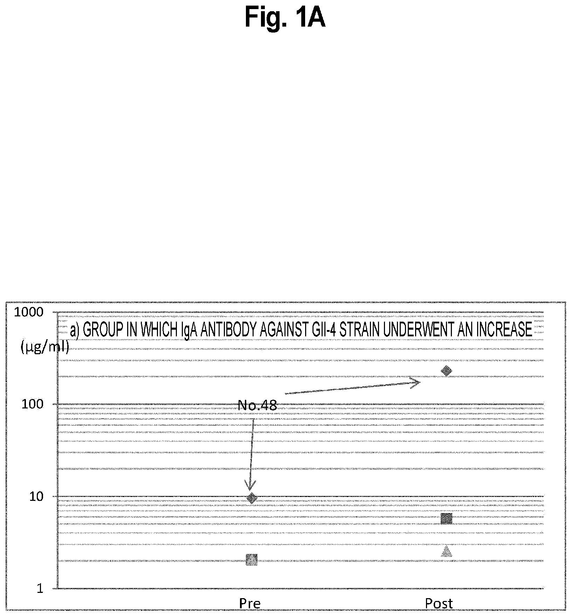

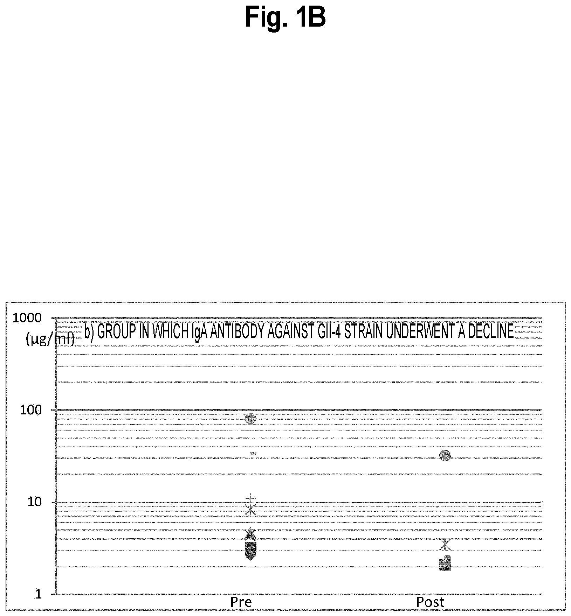

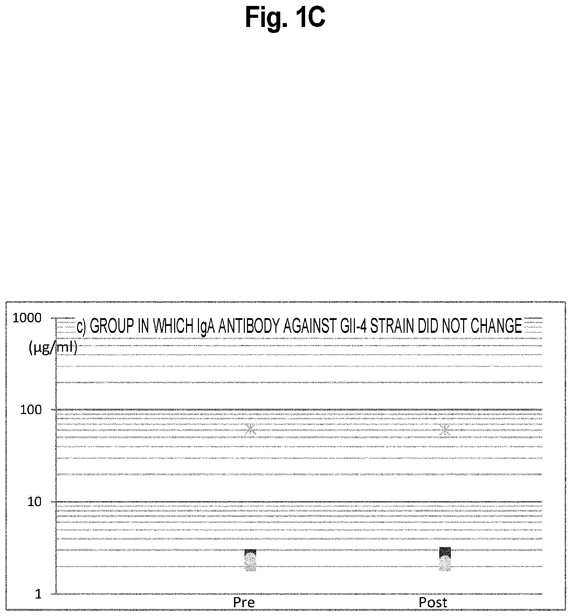

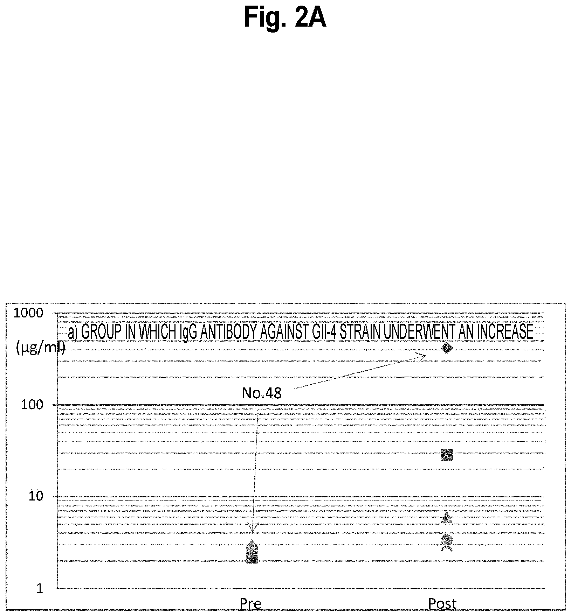

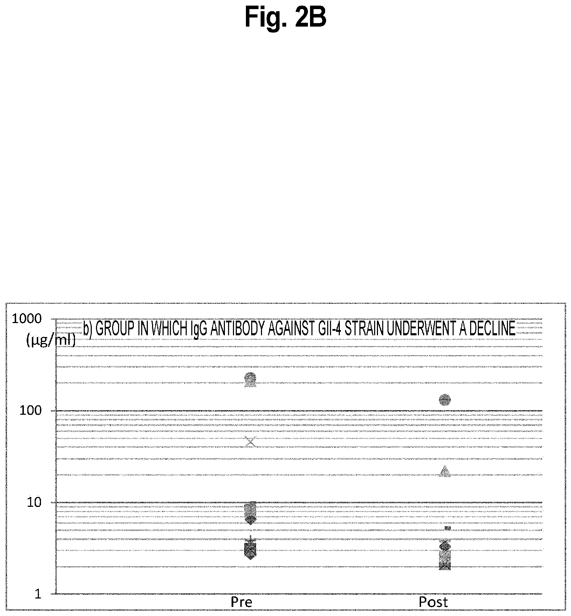

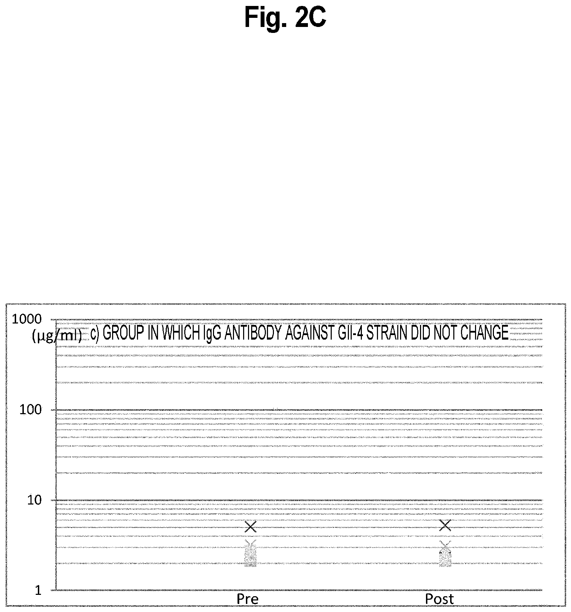

Salivary IgA antibodies to norovirus have quite recently been reported to be deeply involved in norovirus infection, onset, and worsening (Non-Patent Document 1). However, according to the reported results, there are cases who do not develop nor worsen infection even when salivary IgA antibodies are low (Fig. 2), and the interpretation of the risk of infection using IgA antibodies alone is thus necessarily problematic. In addition, the results in Fig. 1 in Non-Patent Document 2 also show examples in which there is no infection by norovirus at a low salivary IgA titer. The conclusion can therefore be drawn that the interpretation of whether IgA antibodies are involved in norovirus infection is problematic.

### Solution to Problem

The present inventors reached the following conclusion and accomplished the present invention as a result: for the prevention of norovirus infection, not only anti-norovirus IgA antibodies (hereinafter also referred to simply as "IgA antibodies") contained in oral cavity-derived samples, but also anti-norovirus IgG antibodies (hereinafter also referred to simply as "IgG antibodies") are involved in the prevention of infection, and actually IgG antibodies may be considered the more important factor in the prevention of infection.

That is, the present application encompasses the following invention.
[1] A method of assisting a determination of whether a subject is susceptible to infection by norovirus or whether a subject is susceptible to post-infection worsening, the method including: a step of measuring, once or a plurality of times, a concentration of anti-norovirus IgG antibody that is present in a sample originating from within the oral cavity of the subject.
[2] The method according to [1], wherein the IgG antibody concentration present in the sample is measured once according to the concentration measurement step, and wherein the method further includes: a step of assessing, when the IgG antibody concentration present in the sample is at least 3 µg/mL, that the subject is relatively resistant to infection by norovirus or is relatively resistant to post-infection worsening.
[3] The method according to [1], wherein the IgG antibody concentration present in the sample is measured once according to the concentration measurement step, and wherein the method further includes: a step of assessing, when the IgG antibody concentration present in the sample is less than 3 µg/mL, that the subject is susceptible to infection by norovirus or is susceptible to post-infection worsening.
[4] The method according to [1], wherein the IgG antibody concentration present in a sample is measured at least twice using the concentration measurement step, and wherein the method further includes: a step of assessing, for the case in which the IgG antibody concentration measured at a certain time point is less than 3 µg/mL and the IgG antibody concentration measured thereafter undergoes an increase with elapsed time, that the subject is susceptible to infection by norovirus or is susceptible to post-infection worsening, and in cases other than this, assessing that the subject is relatively resistant to infection by norovirus or is relatively resistant to post-infection worsening.
[5] The method according to any of [1] to [4], wherein the norovirus is classified into genogroup II (GII).
[6] The method according to [5], wherein the norovirus is a GII-4 strain or a GII-17 strain.
[7] The method according to any of [1] to [6], wherein, in the concentration measurement step, an anti-norovirus IgA antibody concentration is measured together with the measurement of the IgG antibody concentration present in the sample, or wherein the method further includes: a step of measuring an anti-norovirus IgA antibody concentration present in a sample.
[8] A method for evaluating the efficacy of administration of a norovirus vaccine, the method including: a step of measuring an anti-norovirus IgG antibody concentration present in a sample derived from within the oral cavity of a subject.
[9] The method according to [8], including: a step of assessing that a norovirus vaccine is effective when an IgG antibody concentration in a sample that was less than 3 µg/mL prior to administration is increased to at least 3 µg/mL after administration of the norovirus vaccine.
[10] The method according to [8] or [9], wherein the IgG antibody concentration present in samples from a subject to whom a norovirus vaccine has been administered is measured at least twice using the concentration measurement step, and wherein the method further includes: a step of assessing that the norovirus vaccine is effective when the IgG antibody concentration present in a sample from the subject prior to norovirus vaccine administration is less than 3 µg/mL and the IgG antibody concentration measured after administration undergoes an increase to at least 3 µg/mL.
[11] The method according to any of [8] to [10], wherein the norovirus is classified into genogroup II (GII).
[12] The method according to [11], wherein the norovirus is a GII-4 strain or a GII-17 strain.
[13] The method according to any of [8] to [12], wherein, in the concentration measurement step, an anti-norovirus IgA antibody concentration is measured together with the IgG antibody concentration present in the sample, or wherein the method further includes: a step of measuring an anti-norovirus IgA antibody concentration present in a sample.
[14] A method for assisting a determination of whether a norovirus vaccine should be administered, the method including: a step of measuring an anti-norovirus IgG antibody concentration present in a sample derived from within the oral cavity of a subject.
[15] The method according to [14], including: a step of assessing that a norovirus vaccine should be administered when the IgG antibody concentration present in a sample prior to administration of the norovirus vaccine is less than 3 µg/mL.
[16] The method according to [14] or [15], wherein the IgG antibody concentration present in samples is measured at least twice using the concentration measurement step, and wherein the method further includes: a step of assessing, for the case in which the IgG antibody concentration present in a sample after administration of the norovirus vaccine has declined to less than 3 µg/mL, that supplemental norovirus vaccine should be administered.
[17] The method according to any of [14] to [16], wherein the norovirus is classified into genogroup II (GII).
[18] The method according to [17], wherein the norovirus is a GII-4 strain or a GII-17 strain.
[19] The method according to any of [14] to [18], wherein, in the concentration measurement step, an anti-norovirus IgA antibody concentration is measured together with the IgG antibody concentration present in the sample, or wherein the method further includes: a step of measuring an anti-norovirus IgA antibody concentration present in the sample.

### Advantageous Effects of Invention

**The** present invention, by using an IgG antibody concentration as an index, makes it possible to mechanically determine or assess, inter alia, the presence/absence of a norovirus infection risk even without depending on the judgment of a physician.

### Brief Description of Drawings

Fig. 1a gives the results for the group that had an increased concentration of IgA antibody against a GII-4 strain.
Fig. 1b gives the results for the group that had a reduced concentration of IgA antibody against a GII-4 strain.
Fig. 1c gives the results for the group in which the concentration of IgA antibody against a GII-4 strain did not change.
Fig. 2a gives the results for the group that had an elevated concentration of IgG antibody against a GII-4 strain.
Fig. 2b gives the results for the group that had a reduced concentration of IgG antibody against a GII-4 strain.
Fig. 2c gives the results for the group in which the concentration of IgG antibody against a GII-4 strain did not change.
Fig. 3a gives the results for the group that had an elevated concentration of IgA antibody against a GII-17 strain.
Fig. 3b gives the results for the group that had a reduced concentration of IgA antibody against a GII-17 strain.
Fig. 3c gives the results for the group in which the concentration of IgA antibody against a GII-17 strain did not change.
Fig. 4a gives the results for the group that had an elevated concentration of IgG antibody against a GII-17 strain.
Fig. 4b gives the results for the group that had a reduced concentration of IgG antibody against a GII-17 strain.
Fig. 4c gives the results for the group in which the concentration of IgG antibody against a GII-17 strain did not change.

### Description of Embodiments

Embodiments of the present invention (referred to below as the "present embodiments") are described in the following, but the scope of the present invention is not to be interpreted as being limited to or by the following embodiments.

### (Method for Assisting Determination)

In a first aspect, there is provided a method of assisting a determination of whether a subject is susceptible to infection by norovirus or whether a subject is susceptible to post-infection worsening, wherein this method contains a step of measuring, once or a plurality of times, the concentration of anti-norovirus IgG antibody that is present in a sample originating from within the oral cavity of the subject.

The subject may be any mammal for which there is a suspicion of norovirus infection, with humans being the preferred subjects contemplated for the present method.

A sample derived from within the oral cavity of a subject, such as, for example, saliva, sputum, throat swab, mucosal tissue, and so forth, is used as the sample from the standpoint of local immunity. The use of saliva as the sample is preferred from the standpoint of reducing the impact on the subject during collection. Saliva has a clear relationship with antibodies and can be regarded as a preferred sample from this standpoint. Sample collection can be performed by a method known to the individual skilled in the art. For example, in the case of saliva, the subject may directly collect a collection tube containing a prescribed amount of saliva, for example, 1 mL or more.

The collected sample may be further submitted to additional steps prior to measurement of the antibody titer. For example, the collected saliva may be centrifuged and the resulting supernatant may be diluted, followed by measurement of the antibody titer.

The collected sample is submitted to a step of measuring, once or a plurality of time, an antibody concentration. The immunoglobulin that is the target of the measurement may be in the IgG class and may be any of the IgG1, IgG2, IgG3, and IgG4 subclasses or may be a combination thereof.

The norovirus infection-related risk, and specifically the risk of noninfection/infection with norovirus and the risk of nonworsening/worsening due to infection onset, is evaluated using the IgG antibody concentration in the sample as an index. A subject can be assessed as relatively resistant to norovirus infection, or relatively resistant to worsening post-infection, when the IgG antibody concentration in the sample is measured once and the IgG antibody concentration in the sample is greater than or equal to a prescribed concentration, for example, at least 2.7 to 3.3 µg/mL, preferably at least 3 µg/mL, and more preferably at least 3.3 µg/mL. On the other hand, a subject can be assessed as susceptible to norovirus infection, or susceptible to worsening post-infection, when the IgG antibody concentration in the sample is measured once and the IgG antibody concentration in the sample is less than a prescribed value, for example, less than 2.7 to 3.3 µg/mL, preferably less than 3 µg/mL, and more preferably less than 2.7 µg/mL.

The measurement step is preferably carried out a plurality of times because the IgG antibody concentration can change with elapsed time, particularly pre-versus-post-infection. For example, in an embodiment, the sample IgG antibody concentration is measured at least twice using the IgG antibody measurement step. In this case, the subject can be assessed as susceptible to norovirus infection, or susceptible to worsening post-infection, when the IgG antibody concentration measured at a certain time point is less than a prescribed concentration, for example, less than 2.7 to 3.3 µg/mL, preferably less than 3 µg/mL, and more preferably less than 2.7 µg/mL, and the IgG antibody concentration measured after this has increased with elapsed time. In other cases, that is, at or above a prescribed concentration, for example, at least 2.7 to 3.3 µg/mL, preferably at least 3 µg/mL, and more preferably at least 3.3 µg/mL, the assessment can be made that the subject is relatively resistant to norovirus infection or is relatively resistant to worsening post-infection.

A known procedure, for example, an immunological procedure, can be used as the method for measuring the IgG antibody concentration. Immunological procedures can be exemplified by enzyme-linked immunosorbent assays (ELISA); immunostaining (including fluorescent antibody methods, enzyme-antibody methods, heavy metal-labeled antibody methods, and radioisotope-labeled antibody methods); methods that combine electrophoretic separation with a detection method based on, e.g., fluorescence, enzymes, radioisotopes, and so forth (including western blotting and fluorescent two-dimensional electrophoresis); dot blotting; latex agglutination (LA: latex agglutination-turbidimetric immunoassay); and immunochromatography.

As used in the present Description, "norovirus" denotes norovirus of any genotype. Genogroup type I and genogroup type II are examples of norovirus genotypes. While not intended as a limitation, GI-4 strains and so forth are present in the GI type and GII-4 strains and GII-17 strains are present in the GII type.

Components present in the sample besides IgG, and for which a relationship with norovirus infection is known, may be measured in the measurement step; for example, the concentration of anti-norovirus IgA antibody may also be measured in the measurement step. By itself, the IgA antibody concentration does not constitute an index for assessing whether there is a susceptibility to norovirus infection or whether there is a susceptibility to post-infection worsening; however, it can be used in an assistive or auxiliary manner when the IgG antibody concentration is evaluated. In a preferred embodiment, the IgA antibody concentration in the sample is measured in the same step as for the IgG antibody, but a step in which the IgA antibody concentration is separately measured may be additionally provided. IgA antibody participates in suppression of onset of norovirus infection or a prevention of worsening, and there is a suspicion of infection with norovirus for a subject from whom samples have been collected when the sample IgA antibody concentration is increased, and preferably is increased at least 4-fold, from the concentration in a previous measurement.

Assessment of the norovirus infection-related risk can be performed by using the sample IgG antibody concentration in the assistive manner and by a comprehensive diagnosis by a physician from, e.g., the clinical symptoms of the subject, the infection status of the surroundings, and so forth. Known procedures used in the diagnosis of norovirus may be incorporated in and combined with the present method. For example, the detection of norovirus in fecal matter using a test kit is an example of such a known procedure.

Virological diagnostic methods are examples of methods for reliably checking whether a norovirus infection is in progress. For example, since the virus is excreted in large amounts in the fecal matter and vomit of a patient (subject) infected with norovirus, the presence/absence of norovirus in such samples can be confirmed by electron microscopy, reverse transcription PCR, real-time PCR, and so forth.

### (Method for Evaluating Vaccine Efficacy)

In a second aspect, there is provided a method for evaluating the efficacy of administration of a norovirus vaccine, wherein the method contains a step of measuring an anti-norovirus IgG antibody concentration present in a sample derived from within the oral cavity of a subject.

The subject may be any mammal to whom a norovirus vaccine has been administered, with humans being the preferred subjects contemplated for the present method. The norovirus vaccine should recognize a norovirus antigen, but is not otherwise particularly limited. The sample is as has been described in the preceding.

The present method can evaluate the efficacy of administration of a norovirus vaccine using IgG antibody as an index in the same manner as in the method for assisting determination. In a preferred embodiment, a norovirus vaccine can be assessed as effective when the sample IgG antibody concentration after the administration of the norovirus vaccine is increased to at least a prescribed value, for example, at least 2.7 to 3.3 µg/mL, preferably at least 3 µg/mL, and more preferably at least 3.3 µg/mL. For example, a norovirus vaccine is assessed as effective when a sample IgG antibody concentration that was less than 3 µg/mL prior to administration is increased to at least 3 µg/mL after administration of the norovirus vaccine.

The measurement step is preferably carried out a plurality of times because the IgG antibody concentration can change with elapsed time, particularly pre-versus-post-administration of the norovirus vaccine. For example, in an embodiment, the sample IgG antibody concentration is measured at least twice using the IgG antibody measurement step. In this case, the administered norovirus vaccine can be assessed as effective when the concentration of IgG antibody contained in a sample from a subject prior to administration of the norovirus vaccine is less than a prescribed concentration, for example, less than 2.7 to 3.3 µg/mL, preferably less than 3 µg/mL, and more preferably less than 2.7 µg/mL, and the IgG antibody concentration measured after administration has increased to at least a prescribed concentration, for example, at least 2.7 to 3.3 µg/mL, preferably at least 3 µg/mL, and more preferably at least 3.3 µg/mL.

Components present in the sample besides IgG, and for which a relationship with the evaluation of norovirus vaccine efficacy is known, may be measured in the measurement step; for example, the concentration of anti-norovirus IgA antibody may also be measured in the measurement step. By itself, the IgA antibody concentration does not constitute an index for assessing whether there is a susceptibility to norovirus infection or whether there is a susceptibility to worsening post-infection; however, it can be used in an assistive or auxiliary manner when the IgG antibody concentration is evaluated. In a preferred embodiment, the IgA antibody concentration in the sample is measured in the same step as for the IgG antibody, but a step in which the IgA antibody concentration is separately measured may be additionally provided.

The norovirus vaccine may be one against norovirus of any genotype, but a norovirus vaccine against GI type norovirus and/or against GII type norovirus is preferred.

### (Method for Determining Administration Timing)

A third aspect provides a method for assisting a determination of whether a norovirus vaccine should be administered, wherein the method contains a step of measuring an anti-norovirus IgG antibody concentration present in a sample derived from within the oral cavity of a subject.

The subject may be any mammal for which there is suspicion of infection with norovirus, with humans being the preferred subjects contemplated for the present method. The sample is as has been described in the preceding.

The present method can assess, using IgG antibody as an index in the same manner as in the method for assisting determination, whether a norovirus vaccine should be administered. In a preferred embodiment, the assessment can be made that a norovirus vaccine should be administered when the IgG antibody concentration present in a sample from a subject prior to administration of the norovirus vaccine is less than a prescribed value, for example, less than 2.7 to 3.3 µg/mL, preferably less than 3 µg/mL, and more preferably less than 2.7 µg/mL.

The measurement step is preferably carried out a plurality of times because the IgG antibody concentration can change with elapsed time, particularly pre-versus-post-infection. In addition, norovirus, which is in the RNA virus group like seasonal influenza viruses, mutates frequently, and epidemic strains change every one or two years. Due to this, preferably the assessment is made that a supplemental norovirus vaccine should be administered when the IgG antibody concentration is measured before and after vaccine administration and the IgG antibody concentration in the sample post-administration has declined to less than 3 µg/mL. Therefore, for example, in an embodiment, the sample IgG antibody concentration is measured at least twice using the IgG antibody measurement step. The concentration of anti-norovirus IgA antibody may also be measured in the measurement step.

The present invention is specifically described using the examples provided hereinbelow, but the present invention is not limited to or by the examples.

### Examples

### Subjects

The subjects were 106 outpatients at Niigata University Hospital, and cancer patients and patients receiving steroids were excluded. Patients receiving steroids were excluded because the administration of steroids may affect the body's immune system, and it was thought that an increase in antibodies might not occur even in the presence of infection. Cancer patients were also excluded due to the strong possibility that they were receiving drugs that would affect the immune system. The male/female breakdown was as follows: male = 60, female = 46 (median age: 66.5 years).

### Sampling of Saliva Samples

Saliva samples were collected from the subjects in the following intervals.
Pre samples: saliva sampling in October and November, 2018
Post samples: saliva sampling between March and May, 2019

Sampling was performed by the subject himself/herself directly delivering at least 1 mL of saliva into a collection tube. 180 µL of the collected saliva was placed in a centrifugation tube and centrifugation was carried out (10,000 rpm, 5 minutes). 225 µL of PBS (phosphate buffer) was added to 75 µL of the supernatant (4-fold dilution) and a 4-fold dilution series was constructed up to a 256-fold dilution. The antibody titer was measured using each diluted sample. Based on considerations of nonspecific reactions and sensitivity, the antibody titer in the 64-fold diluted sample was ultimately used.

### Measurement of Anti-Norovirus IgA Antibody and IgG Antibody

### Concentrations in the Saliva

The IgA antibody and IgG antibody concentrations in the collected saliva were measured by the ELISA method. The ELISA method was carried out according to the following protocol.

### ELISA Protocol

· GII-4 aomori strain virus-like particles (VLPs (GII-4)) and GII-17 kawasaki strain VLPs (GII-17), expressed using the magnICON (registered trademark) system in tobacco plants, are diluted to a concentration of 1 µg/mL using pH 7.4 PBS (Nacalai Tesque, Inc.).
· 50 µL of each VLP dilution is dripped into each well of a 96-well microplate (Nunc) and standing overnight at 4°C is carried out.
· After standing, the plate is washed three times with pH 7.4 PBS containing 0.1% polyoxyethylene(20) sorbitan monolaurate (WAKO) (PBST).
· After washing, 80 µL of pH 7.4 PBS containing 1% bovine serum albumin (Sigma) (PBSB) is dripped into each well for blocking and incubation is carried out for 2 hours at room temperature.
· The PBSB is discarded.
· 50 µL of the saliva sample diluted 64-fold with PBS is dripped into each well and incubation is carried out for 4 hours at room temperature.
· The plate is washed three times with PBST.
· Into each well is dripped 50 µL of anti-human IgA antibody peroxidase-labeled antibody (goat) (SouthernBiotech) and anti-human IgG antibody peroxidase-labeled antibody (goat) (SouthernBiotech), diluted 2,000-fold with PBSTB, and incubation is carried out for 2 hours at room temperature.
· The plate is then washed five times with PBST.
· After washing, 50 µL of TMB ELISA substrate solution (Thermo Scientific Inc.) is dripped into each well.
· Incubation is carried out at room temperature in the absence of light until color generation can be confirmed.
· 50 µL of STOP solution (Invitrogen) is dripped into each well and the reaction is stopped.
· The absorbance at 450 nm is measured.
· A calibration curve is constructed using humanized IgA antibody and humanized IgG antibody against GII-4 and GII-17 produced by Dr. Takahashi in the Department of Immunology, National Institute of Infectious Diseases of Japan, and the antibody concentration is determined from the absorbance of the sample.

The ELISA results are given in Figs 1 to 4.

### Change in Concentrations of Salivary IgA Antibody and IgG Antibody Against a GII-4 Strain

According to the results of a comparison of the IgA antibody concentrations and IgG antibody concentrations in the Pre samples with the IgA antibody concentrations and IgG antibody concentrations in the Post samples, classification into the following was possible for both the IgA antibody and IgG antibody: a group in which the antibody concentration in the Pre sample is increased in the Post sample; a group in which the antibody concentration in the Pre sample is reduced in the Post sample; and a group in which the antibody concentration in the Pre sample is almost unchanged in the Post sample (Fig. 1).

Fig. 1 gives the results of a comparison of the concentrations of salivary IgA antibody against the GII-4 strain in the Pre samples and Post samples. This is a distribution diagram for the IgA antibody concentrations in the Pre and Post samples from each of the volunteers into a) a group in which the IgA antibody concentration increased, b) a group in which the IgA antibody concentration declined, and c) a group in which the IgA antibody concentration did not change.

In the case of infection with, e.g., a virus, the concentration of antibody against the infecting virus generally increases. With the group in which the IgA antibody concentration increased (Fig. 1a)), there is a suspicion of some sort of infection with the GII-4 strain in the Pre/Post sampling interval. In addition, with the declining group (Fig. 1b)), it is thought that there was no infection in the sampling interval.

When the IgA antibody concentration distribution for Pre for the group in which the IgA antibody concentration increased is compared with the IgA antibody concentration distribution for Pre for the group in which the IgA antibody concentration declined, the IgA antibody concentration distribution for the declining group is distributed to higher concentrations. It is thus thought that there is a susceptibility to GII-4 strain infection in the case of a low IgA antibody concentration, but there is a relative resistance to infection in the case of a high IgA antibody concentration.

Volunteer No. 48 experienced the onset of symptoms such as vomiting (wife also developed symptoms at a later date), and there was a strong suspicion of norovirus infection. However, the Pre IgA antibody concentration for No. 48 is high at 10 µg/mL and is the same as the Pre IgA antibody concentration distribution for the declining group, but there is a strong suspicion of infection. The prevention of infection cannot be explained by salivary IgA antibodies alone.

Fig. 2 shows the results of a comparison of the concentrations of salivary IgG antibody against the GII-4 strain for the Pre samples and Post samples. This is a distribution diagram for the IgG antibody concentrations in the Pre and Post samples from each of the volunteers into a) a group in which the IgG antibody concentration increased, b) a group in which the IgG antibody concentration declined, and c) a group in which the IgG antibody concentration did not change.

With the group in which the IgG antibody concentration increased (Fig. 2a)), as with Fig. 1 there is a suspicion of some sort of infection with the GII-4 strain in the Pre/Post sampling interval. In addition, with the declining group (Fig. 2b)), it is thought that there was no infection in the sampling interval, and this is thought to be a group in which there was infection prior to sampling.

When the IgG antibody concentration distribution for Pre for the group in which the IgG antibody concentration increased is compared with the IgG antibody concentration distribution for Pre for the group in which the IgG antibody concentration declined, as with the IgA antibody concentrations in Fig. 1, the IgG antibody concentration distribution for the declining group is distributed to higher concentrations. Unlike the IgA antibody concentrations, in the case of the IgG antibody concentrations, the IgG antibody concentration for Pre for the increasing group was distributed at less than 3 µg/mL, while the IgG antibody concentration for Pre in the declining group was distributed to 3 µg/mL and above. Thus, it is thought that there is infection by the GII-4 strain when the IgG antibody concentration is less than 3 µg/mL and there is no infection at 3 µg/mL and above. A correlation between the change in IgG antibody concentration and the change in IgA antibody concentration could not be confirmed.

The IgA antibody concentration for Pre for volunteer No. 48, for whom infection was strongly suspected, was high at 10 µg/mL, and this was the same for the IgA antibody concentration distribution for Pre for the declining group; however, the IgG antibody concentration for Pre for No. 48 was clearly lower than the IgG antibody concentration distribution for the declining group. It was thought, based on this result, that not only salivary IgA antibodies but also IgG antibodies are involved with the prevention of infection, and actually that IgG antibodies are the more important factor for infection prevention.

### Change in Concentrations of Salivary IgA Antibody and IgG Antibody Against a GII-17 Strain

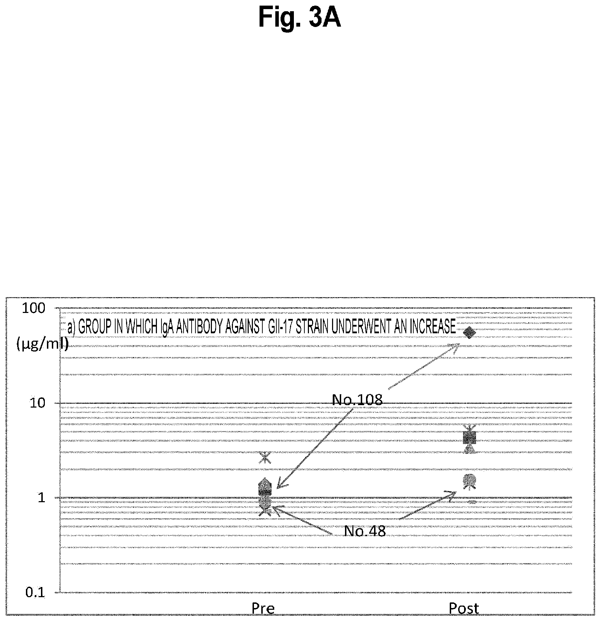

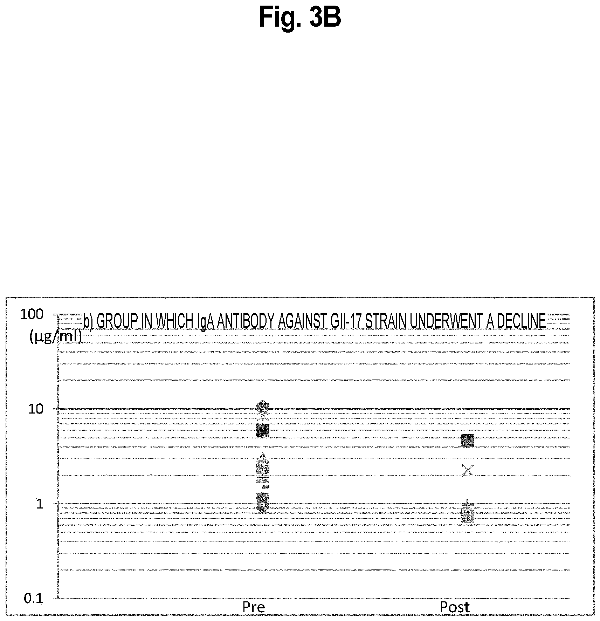

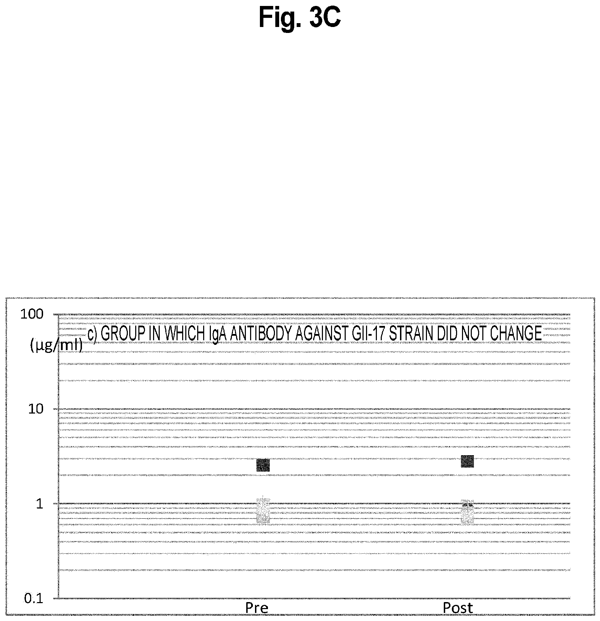

As for the results against the GII-4 strain, according to the results of a comparison of the IgA antibody concentrations and IgG antibody concentrations in the Pre samples with the IgA antibody concentrations and IgG antibody concentrations in the Post samples, classification into the following was possible for both the IgA antibody and IgG antibody: a group in which the antibody concentration in the Pre sample is increased in the Post sample; a group in which the antibody concentration in the Pre sample is reduced in the Post sample; and a group in which the antibody concentration in the Pre sample is almost unchanged in the Post sample (Fig. 3).

Fig. 3 gives the results of a comparison of the concentrations of salivary IgA antibody against the GII-17 strain in the Pre samples and Post samples. This is a distribution diagram for the IgA antibody concentrations in the Pre and Post samples from each of the volunteers into a) a group in which the IgA antibody concentration increased, b) a group in which the IgA antibody concentration declined, and c) a group in which the IgA antibody concentration did not change.

When the IgA antibody concentration distribution for Pre for the group in which the IgA antibody concentration increased is compared with the IgA antibody concentration distribution for Pre for the group in which the IgA antibody concentration declined, the IgA antibody concentration distribution for the declining group is distributed to higher concentrations, but the IgA antibody concentration distributions in the two groups partially overlap. It is thus thought that there is a susceptibility to GII-17 strain infection in the case of a low IgA antibody concentration, but there is a relative resistance to infection in the case of a high IgA antibody concentration.

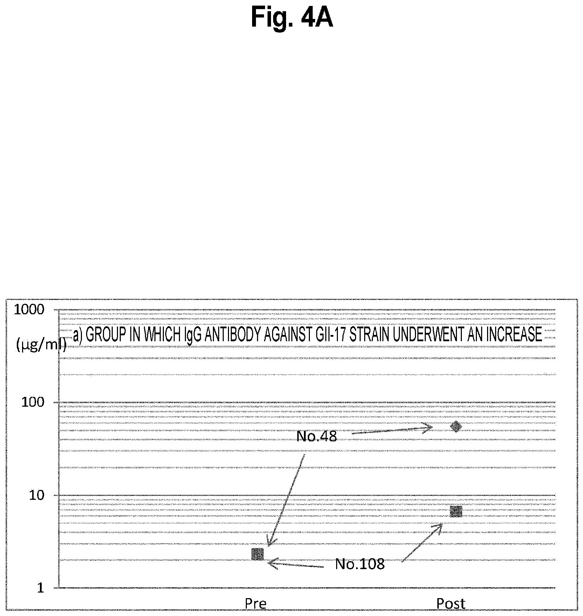

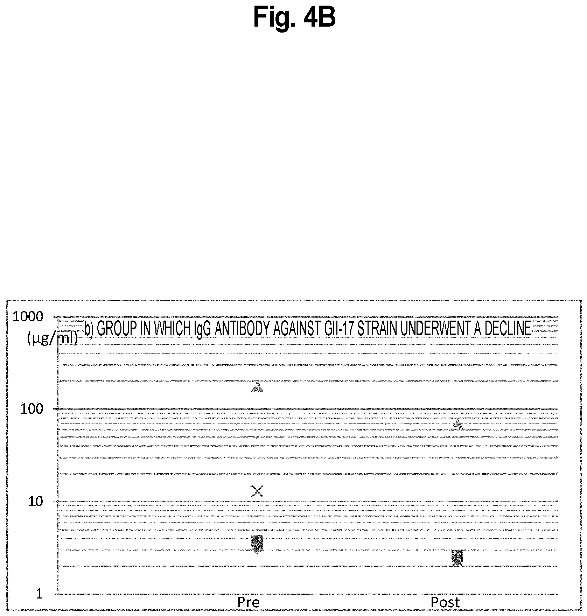

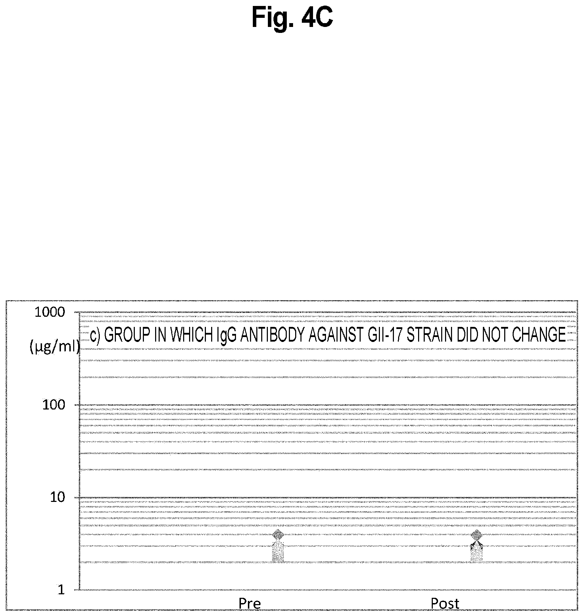

Fig. 4 shows the results of a comparison of the concentrations of salivary IgG antibody against the GII-17 strain for the Pre samples and Post samples. This is a distribution diagram for the IgG antibody concentrations in the Pre and Post samples from each of the volunteers into a) a group in which the IgG antibody concentration increased, b) a group in which the IgG antibody concentration declined, and c) a group in which the IgG antibody concentration did not change.

With the group in which the IgG antibody concentration increased (Fig. 4a)), as with Fig. 3 there is a suspicion of some sort of infection with the GII-17 strain in the Pre/Post sampling interval. In addition, with the declining group (Fig. 4b)), it is thought that there was no infection in the sampling interval.

When the IgG antibody concentration distribution for Pre for the group in which the IgG antibody concentration increased is compared with the IgG antibody concentration distribution for Pre for the group in which the IgG antibody concentration declined, as above the IgG antibody concentration distribution for the declining group is distributed to higher concentrations. Unlike the IgA antibody concentrations, and as in Fig. 2, in the case of the IgG antibody concentrations, the IgG antibody concentration for Pre for the increasing group was distributed at less than 3 µg/mL, while the IgG antibody concentration for Pre in the declining group was distributed to 3 µg/mL and above. Thus, it is thought that there is a susceptibility to infection by the GII-17 strain when the IgG antibody concentration is less than 3 µg/mL and there is a relative resistance to infection when the IgG antibody concentration is at least 3 µg/mL.

The IgA antibody concentration for Pre for volunteer No. 48, for whom infection was strongly suspected (for No. 108, there was also a suspicion of infection, but no symptoms were present), was the same as the IgA antibody concentration distribution for Pre for the declining group; however, the IgG antibody concentration for Pre for No. 48 was clearly lower than the IgG antibody concentration distribution for the declining group. It was thought that not only salivary IgA antibodies but also IgG antibodies are involved with the prevention of infection, and actually that IgG antibodies are the more important factor for infection prevention.

## Claims

1. A method of assisting a determination of whether a subject is susceptible to infection by norovirus or whether a subject is susceptible to post-infection worsening, the method comprising:
a step of measuring, once or a plurality of times, a concentration of anti-norovirus IgG antibody that is present in a sample originating from within the oral cavity of the subject.

2. The method according to claim 1, wherein the IgG antibody concentration present in the sample is measured once according to the concentration measurement step, and wherein the method further comprises:
a step of assessing, when the IgG antibody concentration present in the sample is at least 3 µg/mL, that the subject is relatively resistant to infection by norovirus or is relatively resistant to worsening post-infection.

3. The method according to claim 1, wherein the IgG antibody concentration present in the sample is measured once according to the concentration measurement step, and wherein the method further comprises:
a step of assessing, when the IgG antibody concentration present in the sample is less than 3 µg/mL, that the subject is susceptible to infection by norovirus or is susceptible to worsening post-infection.

4. The method according to claim 1, wherein the IgG antibody concentration present in a sample is measured at least twice using the concentration measurement step, and wherein the method further comprises:
a step of assessing, for the case in which the IgG antibody concentration measured at a certain time point is less than 3 µg/mL and the IgG antibody concentration measured thereafter undergoes an increase with elapsed time, that the subject is susceptible to infection by norovirus or is susceptible to worsening post-infection, and in cases other than this, assessing that the subject is relatively resistant to infection by norovirus or is relatively resistant to worsening post-infection.

5. The method according to any one of claims 1 to 4, wherein the norovirus is classified into genogroup II (GII).

6. The method according to claim 5, wherein the norovirus is a GII-4 strain or a GII-17 strain.

7. The method according to any one of claims 1 to 6, wherein, in the concentration measurement step, an anti-norovirus IgA antibody concentration is measured together with the measurement of the IgG antibody concentration present in the sample, or wherein the method further comprises:
a step of measuring an anti-norovirus IgA antibody concentration present in a sample.

8. A method for evaluating the efficacy of administration of a norovirus vaccine, the method comprising:
a step of measuring an anti-norovirus IgG antibody concentration present in a sample derived from within the oral cavity of a subject.

9. The method according to claim 8, comprising:
a step of assessing that a norovirus vaccine is effective when an IgG antibody concentration in a sample that was less than 3 µg/mL prior to administration is increased to at least 3 µg/mL after administration of the norovirus vaccine.

10. The method according to claim 8 or 9, wherein the IgG antibody concentration present in samples from a subject to whom a norovirus vaccine has been administered is measured at least twice using the concentration measurement step, and wherein the method further comprises:
a step of assessing that the norovirus vaccine is effective when the IgG antibody concentration present in a saliva sample from the subject prior to norovirus vaccine administration is less than 3 µg/mL and the IgG antibody concentration measured after administration undergoes an increase to at least 3 µg/mL.

11. The method according to any one of claims 8 to 10, wherein the norovirus is classified into genogroup II (GII).

12. The method according to claim 11, wherein the norovirus is a GII-4 strain or a GII-17 strain.

13. The method according to any one of claims 8 to 12, wherein, in the concentration measurement step, an anti-norovirus IgA antibody concentration is measured together with the IgG antibody concentration present in the sample, or wherein the method further comprises:
a step of measuring an anti-norovirus IgA antibody concentration present in a sample.

14. A method for assisting a determination of whether a norovirus vaccine should be administered, the method comprising:
a step of measuring an anti-norovirus IgG antibody concentration present in a sample derived from within the oral cavity of a subject.

15. The method according to claim 14, comprising:
a step of assessing that a norovirus vaccine should be administered when the IgG antibody concentration present in a sample prior to administration of the norovirus vaccine is less than 3 µg/mL.

16. The method according to claim 14 or 15, wherein the IgG antibody concentration present in samples is measured at least twice using the concentration measurement step, and wherein the method further comprises:
a step of assessing, for the case in which the IgG antibody concentration present in a sample after administration of the norovirus vaccine has declined to less than 3 µg/mL, that supplemental norovirus vaccine should be administered.

17. The method according to any one of claims 14 to 16, wherein the norovirus is classified into genogroup II (GII).

18. The method according to claim 17, wherein the norovirus is a GII-4 strain or a GII-17 strain.

19. The method according to any one of claims 14 to 18, wherein, in the concentration measurement step, an anti-norovirus IgA antibody concentration is measured together with the IgG antibody concentration present in the sample, or wherein the method further comprises:
a step of measuring an anti-norovirus IgA antibody concentration present in the sample.
